# EUROPEAN PATENT SPECIFICATION

(11) **EP 1 488 008 B1**
(45) Date of publication and mention of the grant of the patent: **16.07.2008**
(21) Application number: 03744855.2
(22) Date of filing: 25.03.2003
(51) Int. Cl.: C12Q 1/68

(54) **METHOD FOR DNA METHYLATION ANALYSIS**
VERFAHREN ZUM NACHWEIS VON DNA METHYLIERUNG
PROCEDE POUR ANALYSER LA METHYLATION DE L'ADN

(30) Priority: 25.03.2002 DE 10214232
(43) Date of publication of application: 22.12.2004
(73) Proprietor: Epigenomics AG, 10178 Berlin (DE)
(72) Inventor: BERLIN, Kurt, 14532 Stahnsdorf (DE)
(74) Representative: Schubert, Klemens
(86) International application number: PCT/EP2003/003104
(87) International publication number: WO 2003/080862

(56) References cited:
- WO-A-02/18632
- WO-A-92/15674
- WO-A-99/10540
- US-A- 6 140 110
- PRADHAN SRIHARSA ET AL: "Recombinant human DNA (cytosine-5) methyltransferase: I. Expression, purification, and comparison of de novo and maintenance methylation." JOURNAL OF BIOLOGICAL CHEMISTRY, vol. 274, no. 46, 12 November 1999 (1999-11-12), pages 33002-33010, XP002248163 ISSN: 0021-9258 cited in the application
- REIN ET AL: "Identifying 5-methylcytosine and related modifications in DNA genomes" NUCLEIC ACIDS RESEARCH, OXFORD UNIVERSITY PRESS, SURREY, GB, vol. 26, no. 10, 1998, pages 2255-2264, XP002143106 ISSN: 0305-1048
- SZYF M: "THE DNA METHYLATION MACHINERY AS A THERAPEUTIC TARGET" CURRENT DRUG TARGETS, BENTHAM SCIENCE PUBLISHER,, US, vol. 1, no. 1, July 2000 (2000-07), pages 101-118, XP001122812 ISSN: 1389-4501
- HERMAN J G ET AL: "METHYLATION-SPECIFIC PCR: A NOVEL PCR ASSAY FOR METHYLATION STATUS OF CPG ISLANDS" PROCEEDINGS OF THE NATIONAL ACADEMY OF SCIENCES OF USA, NATIONAL ACADEMY OF SCIENCE. WASHINGTON, US, vol. 93, 1 September 1996 (1996-09-01), pages 9821-9826, XP002910406 ISSN: 0027-8424

## Description

### Field of the invention

The analysis of methylation of genomic DNA is proving to be of increasing importance. Aberrant genomic methylation patterns have been shown to be implicated in a wide range of disease conditions, including cancer. DNA methylation analysis requires the development of a range of tools specific to the detection of DNA methylation as conventional techniques such as PCR and sequencing are not capable of distinguishing 5-methyl cytosine from unmethylated cytosine.

### Prior Art

The most common covalent modification of genomic DNA is the methylation of cytosine to 5 methyl cytosine. In eukaryotic cell systems the bulk of methylation activity takes place during the S phase of the cell cycle. Complex tissue specific methylation patterns established during development are preserved in newly replicated DNA by the action of maintenance methyltransferases. These enzymes act to methylate genomic DNA that has been semiconservatively replicated. The methyl transfer reaction proceeds through a non specific binding of the transferase to the hemimethylated DNA strand, identification of the target base followed by the recruitment of the methyl donor group, most commonly S-adenosyl-L-methionine (AdoMet) to the active site. DNA methyltransferases (m5C Mtase) attach a methyl group to the 5 position carbon. The reaction is carried out via a covalent intermediate between the enzyme and the base whereby the target cytosine is flipped through 180 degrees. The mechanism of methyltransferase dependant cytosine methylation is further reviewed in articles such as Cheng and Roberts 'AdoMetdependant methylation, DNA methyltransferases and base flipping' Nucleic Acids Res. 15; 29 (18):3784-95.

Several species of methyltransferases have been identified, of particular interest to this invention are the family of maintenance methyltransferases that propagate the methylation pattern of hemimethylated DNA within the unmethylated strand, such as Dnmt1. The in vitro action mechanism of DNMT1 is fully discussed in Pradhan, S., Bacolla, A., Wells, R. D., Roberts, R. J.. 'Recombinant Human DNA (Cytosine-5) Methyltransferase. I. Expression, Purification and comparison of de novo and maintenance methylation.' J. Biol. Chem. 274: 33002-33010 and Bacolla A, Pradhan S, Roberts RJ, Wells RD.'Recombinant human DNA (cytosine-5) methyltransferase. II. Steady-state kinetics reveal allosteric activation by methylated DNA' J Biol Chem. 12; 274 (46) : 33011-9.

Cytosine methylation plays an important role in gene expression and regulation and has been shown to be critical in the maintenance of normal cellular functions. It is associated with genomic imprinting, embryonic development and a wide variety of diseases, including cancer.

For example, aberrant DNA methylation within CpG islands is common in human malignancies leading to abrogation or overexpression of a broad spectrum of genes (Jones, P.A. Cancer Res 65:2463-2467, 1996). Abnormal methylation has also been shown to occur in CpG rich regulatory elements in intronic and coding parts of genes for certain tumours (Chan, M.F., et al., Curr Top Microbiol Immunol 249:75-86,2000). Using restriction landmark genomic scanning, Costello and coworkers were able to show that methylation patterns are tumour-type specific (Costello, J. F., et al., Nat Genet 24:132-138, 2000). Highly characteristic DNA methylation patterns could also be shown for breast cancer cell lines (Huang, T. H.-M., et al., Hum Mol Genet 8:459-470, 1999). Genome wide assessment of methylation status represents a molecular fingerprint of cancer tissues.

Therefore it follows that the analysis of methylation patterns within genomic DNA is of considerable importance. However, 5-methylcytosine analysis currently cannot be carried out using standard molecular biological tool. 5-methylcytosine has the same base pairing behaviour as unmethylated cytosine therefore it cannot be identified by sequencing. Moreover, the epigenetic information carried by 5-methylcytosine is completely lost during PCR amplification. Current methods of analysis are generally carried out using one of two methods. Firstly, methylation sensitive restriction enzyme digest, and secondly by the more versatile technique of bisulphite treatment followed by PCR analysis.

Bisuphite treatment followed by alkaline hydrolysis converts cytosine within a nucleic acid sample to uracil. The treatment is highly specific in that 5-methylcytosine remains unconverted. Thus PCR amplification of the treated DNA results in the synthesis of amplificate nucleic acids wherein thymine is substituted for unmethylated cytosine within the original genomic sequence. The bisulphite treatment is often carried out on minute quantities of genomic DNA which may be lost during handling. The sensitivity of the technique is improved by use of an agarose matrix within which the DNA is enclosed thus preventing the diffusion and renaturation of the DNA (bisulfite only reacts with single-stranded DNA), and which replaces all precipitation and purification steps with fast dialysis (Olek A, Oswald J, Walter J. A modified and improved method for bisulphite based cytosine methylation analysis. Nucleic Acids Res. 1996 Dec 15:24(24):5064-6).

In practice the utility of the bisulphite treatment is often limited by the sensitivity of the technique to small samples. Furthermore, the requirement of the agarose step in order to limit DNA loss reduces the suitability of the technique to automatization.

Methods for the amplification of specific DNA targets are based upon template directed primer extension by polymerases. The most widely utilised of these methods is the polymerase chain reaction 'PCR' (Mullis, K. et al., Cold Spring Harbor Symp. Quant. Biol. 51:263-273 (1986); Erlich H. et al., EP 50,424; EP 84,796, EP 258,017, EP 237,362; Mullis, K., EP 201,184; Mullis K. et al., U.S. Pat. No. 4,683,202; Erlich, H., U.S. Pat. No. 4,582,788; Saiki, R. et al., U.S. Pat. No. 4,683,194 and Higuchi, R."PCR Technology," Ehrlich, H. (ed.), Stockton Press, NY, 1989, pp 61-68).

In the polymerase chain reaction successive cycles of denaturation are followed by annealing and polymerisation. In the first step the DNA double helix is denatured by transient heating. This is followed by the annealing of two species of primers, one to each strand of DNA. Subsequently the annealed primers are extended using a polymerase. This is followed by the denaturation of the resultant double stranded nucleic acids, allowing each strand to serve as a template for another cycle of template directed primer extension.

The polymerase chain reaction (hereafter PCR) is most commonly performed in disposable reaction tubes such as small, plastic microcentrifuge tubes or test tubes which are placed in an instrument containing a thermally controlled heat exchanger. Examples of these instruments are disclosed in U.S. Pat. No. 5,038,852, U.S. application Ser. No. 07/709,374, filed Jun. 3, 1991, and U.S. application Ser. No. 07/871,264, filed Apr. 20, 1992. Alternative devices for the PCR analysis of nucleic acids wherein the reaction is carried out in capillary tubes have been described in U.S. Pat. No. 5,779,977 .

The heat exchanger in these instruments is typically a metal block; however, hot air ovens and water baths also have been used. The temperature of the reaction mixture in the reaction tubes is changed in a cyclical fashion to cause denaturation, annealing and extension reactions to occur in the mixture. Three separate incubation temperatures commonly were used in the first generation PCR thermal cycling applications. These were typically around 94.degree. C. for denaturation, around 55.degree. C. for annealing, and around 72.degree. C. for extension. More recently, the annealing and extension incubations have frequently been combined to yield a two temperature incubation process, typically around 94.degree. C. for denaturation, and around 50.degree.-65.degree. C. for an annealing and extension incubation. The optimal incubation temperatures and times differ, however, with different targets and primer oligonucleotides.

### Description

The invention relates to a method and devices for the enzymatic amplification of nucleic acids whereby the methylation pattern of said nucleic acid is conserved in the amplificate sequence. The method presents improvements over the basic polymerase chain reaction in that a further methylation step is carried out thereby conserving the complex methylation pattern of a genomic DNA in the amplificate nucleic acids. Subsequent to each cycle of the polymerase chain reaction, the hemimethylated nucleic acid is contacted with a maintenance methyltransferase thereby allowing for the methylation of the unmethylated strand of the nucleic acid. Methylation of the hemimethylated DNA by a maintenance methyltransferase is such that the specific methylation pattern of the CpG dinucleotides within the template strand of the nucleic acid is replicated in the unmethylated strand. The described invention thereby allows for the preservation of complex genomic methylation patterns within amplificate nucleic acids.

The maintenance of complex methylation patterns within nucleic acid amplificates allows for the analysis of samples using a variety of methylation specific techniques. Such techniques, which include bisulphite analysis and methylation sensitive restriction enzyme digest were heretofore carried out using limited amounts of DNA samples and/or in combination with a polymerase chain reaction.

### Definitions

The following terms and phrases as used herein are intended to have the meanings as described below.

In the context of the present invention the term 'methylation pattern' is taken to mean the specific consecution of 5 methyl cytosine within a nucleic acid sequence.

In the context of the present invention the term 'template nucleic acid' refers to a single stranded nucleic acid which serves as a template for the synthesis of a nucleic acid from a primer oligonucleotide.

In the context of the present invention the term 'synthesised nucleic acid' is taken to mean a nucleic acid which is the product of a template directed primer extension reaction.

The invention comprises method and devices for the methylation pattern retaining enzymatic amplification of nucleic acids. The method comprises a modified form of the polymerase chain reaction, wherein an additional methylation step during each cycle of the reaction allows for the replication of complex methylation patterns present within a genomic DNA sample in amplificate nucleic acids. The method comprises four steps of which the first three steps are known in the art, for example, in US patents numbers 4,683,195, 4,683,202, and 4,800,159. The fourth step is the novel methylation retaining step.

It is one object of the present invention to provide a method for the amplification of genomic DNA whereby the cytosine methylation pattern of the genomic DNA is retained in the amplificate sequence(s), said method comprising the following steps:
(a) heating the genomic DNA to a temperature operative to cause denaturation
(b) cooling the denatured DNA in the presence of single stranded oligonucleotide primers such that the primers anneal to the DNA
(c) heating the mixture in the presence of polymerase and nucleotides to a temperature such that the primers are extended resulting in a hemimethylated double stranded nucleic acid
(d) contacting the double stranded nucleic acid with a maintenance methyltransferase and a methyl donor molecule
(e) enzymatically methylating the hemimethylated double stranded DNA according to the methylation status of the CpG dinucleotides within the template strand
f) repeating steps A-E a desired number of times to reach a desired number of nucleic acids.

In a preferred embodiment of the present invention the methyltransferase is a maintenance methyltransferase. In a further preferred embodiment the methyltransferase is DNA (cytosine-5) Methyltransferase (DNMT 1).

According to the invention it is preferred that the methyl donor molecule is S-adenosylmethionine.

It is also preferred according to the invention that the methyl group carries a detectable label which is incorporated into the synthesised nucleic acid strand.

In a further preferred embodiment of the present invention a plurality of primer oligonucleotides are immobilised on a solid surface.

It is also preferred that the methyltransferase is immobilised on a solid surface.

According to the invention it is also preferred that the polymerase is immobilised on a solid surface.

In another preferred embodiment of the present invention a further step (g) is present that is a treatment with an agent capable of distinguishing between methylated and unmethylated cytosine bases. Hereby it is preferred that the agent is a methylation sensitive restriction enzyme. It is especially preferred according to the invention that the agent is a bisulphite solution.

Another object of the invention is the use of a device comprising two or more reaction chambers, channel means providing fluid connections between adjacent chambers and the first and last reaction chambers, temperature regulating means for controlling the temperature of each reaction chamber for the methylation pattern retaining amplification of nucleic acids according to Claim 1.

It is preferred according to the invention that the device comprises two vessels, a reaction chamber, temperature regulating means for controlling the temperature of the reaction chamber, means for transferring liquid reagent form the first and second vessels to the reaction chamber, channel means providing fluid connections between adjacent chambers and the first and last reaction chambers means for draining liquid reagents from the reaction chamber for the methylation pattern retaining amplification of nucleic acids.

A nucleic acid obtained according to the method of the invention by optionally using the device according to the invention.

A further object of the present invention is a method of manufacturing a methylated nucleic acid according to the method of the invention by optionally using the device according to the invention.

The details of method of the present invention are as follows.

In the first step of the method (hereinafter referred to as Step A) the sample DNA is heat denatured allowing the single stranded DNA to be used in the analysis, suitable melting temperatures are dependant upon several variables including GC content of the sequence and length of sequence, but in general may be 95°C or higher for 15 seconds to 2 minutes. In the second step (hereinafter referred to as Step B) oligonucleotide primers are annealed to the template sequence at a lower temperature, (typically between 40°C and 60°C for 30 to 60 seconds), again dependant upon the GC content and length of the primers. The oligonucleotides are able to form stable associations ('anneal') with the single stranded DNA (hereinafter referred to as the template strand) and thus serve as primers for nucleic acid synthesis by a DNA polymerase. In the third step (hereinafter referred to as Step C) a corresponding nucleic acid strand to the template is synthesised from the primer oligonucleotide by means of the polymerase and deoxynucleotide triphosphates (dNTPs). The temperature is raised to an optimum for the polymerase, which in the case of commonly used thermostable polymerases is approximately 74°C, primer extension then lasts approximately 1 to 2 minutes. These steps are all common to the polymerase chain reaction. Reactions take place in a mixture which includes a sample of the target DNA, a thermostable DNA polymerase, oligonucleotide primers, deoxynucleotide triphosphates (dNTPs), reaction buffer, magnesium and optional additives. Reaction temperatures and times must be optimised according to factors including, for example, GC content and length of sequence and primers.

Subsequent to enzymatic amplification, the resultant hemimethylated nucleic acid is enzymatically methylated according to the methylation status of the CpG dinucleotides within the template strand. The enzyme should be a maintenance methyltransferase with an affinity for hemimethylated DNA.

This step is executed a user defined number of times. Each repetition of Steps A-E results in a doubling of the number of nucleic acid molecules within the sample. The exponential increase in the quantity of synthesised nucleic acid enables the production of sufficient quantities of methylated nucleic acids for use in other methylation specific analysis techniques such as methylation sensitive restriction enzyme analysis and bisulphite treatment with a greatly increased efficiency.

Suitable methylation enzymes for use in Step D of the method are limited to those capable of methylating the cytosine at the 5 position according to the methylation status of the cytosine within the corresponding CpG dinucleotide on the template strand. In the case of a cytosine within a CpG upon the template strand being methylated, then the corresponding CpG to which it is hybridised on the synthesised strand will be methylated by action of the enzyme at the 5 position of the cytosine base. If the cytosine within said CpG is unmethylated then the corresponding CpG on the synthesised strand will remain unmethylated. The reaction is carried out using appropriate buffers and other reagents and reaction conditions as recommended by the supplier of the enzyme, this may include a methyl donor molecule such as, but not limited to S-adenosylmethionine. Furthermore said methyl group may carry a detectable label, for example a fluorescent label.

The enzyme may be from any source e.g. Human, Mouse, recombinant. In a preferred embodiment the enzyme is DNA Methyltransferase 1 (DNMT1). In a further preferred embodiment the methyltransferase is immobilised upon a solid surface.

The invention further relates to the use of devices for the methylation retaining amplification of a DNA sequence. Several embodiments of devices for the methylation retaining PCR of nucleic acids are envisioned. All devices described herein are controlled by a user defined protocol implemented by a programmable computer. The computer is capable of controlling all variables of the system including sample handling, flow, velocity, pressure, and temperature. Differences between said embodiments arise due to the different means of heating and cooling the reaction mixture and also the alternative means employed for contacting the reagents.

Three embodiments of such devices are described below.

### Multiple reaction chamber methylation retaining PCR device

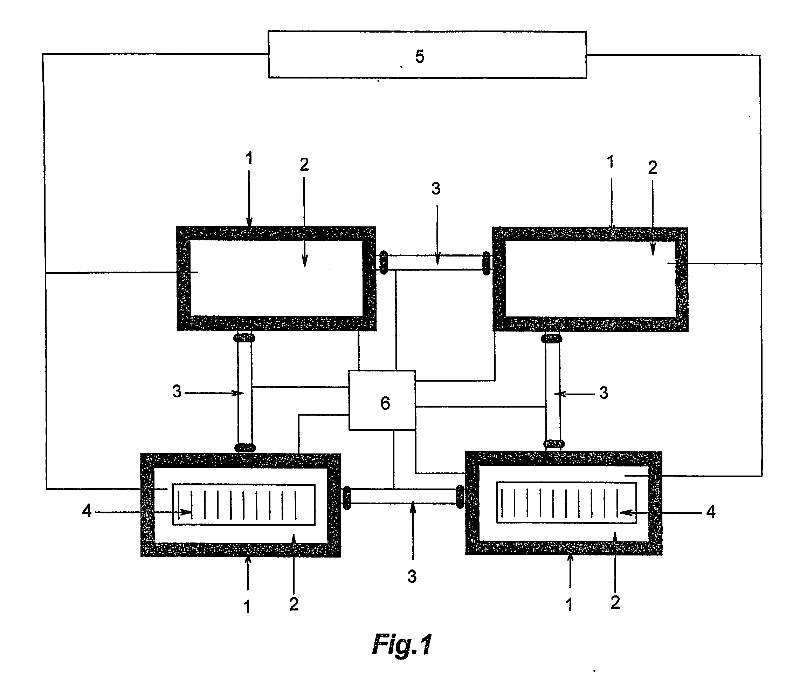

A first embodiment of a methylation retaining PCR device is illustrated in Figure 1, said embodiment is hereinafter referred to as Device 1. The illustrated embodiment has four thermostated units. Each unit comprises a means for temperature maintenance (1), reaction chamber for containing the reaction solution (2), said reaction chambers connected by tube means (3) for transporting the reaction solution between the reaction chambers and further comprising means for introducing additional reagents into the reaction solution. Two of the reaction chambers may further each comprise a solid support (4) upon which is immobilised the enzymatic means for carrying out Steps C or D of the reaction respectively. In a further preferred embodiment the reaction chamber within which Step C is carried out does not further comprise a solid surface up on which the polymerase is immobilised, the polymerase is a component of the reaction solution. The reaction chambers are each connected by tube means in order to transport the reaction solution between each chamber. Transport through the tubes is carried out by means of a pump(6), preferably a peristaltic pump. The tubes further comprise valves ensuring that the reaction solution flows in a unidirectional manner. In a further preferred embodiment the pump mechanism may be a plunger and seal arrangement, similar to a syringe. Said pump may be connected to a first reaction chamber within which the initial denaturation reaction takes placer. The pump mechanism is under control of a computer programmed to carry out the PCR protocol. The pump or plunger is activated back and forth to draw reaction mixture into the reaction chamber,

In a first reaction chamber, Step A of the reaction is carried out whereby the methylated nucleic acid sample is heat denatured in a reaction solution further containing necessary buffers and reagents. Said reaction chamber is kept at a consistent suitable temperature, generally between 85°C and 95°C. The reaction mixture is then passed to the second reaction chamber in order to carry out Step B of the reaction. The second reaction chamber is kept at a consistent temperature suitable for the annealing of primer oligonucleotides to the target DNA. Said temperature is dependant upon the composition of the primers but a general estimate may be calculated using the formula Tm = 81.5 + 16.6 x (log10[Na+]) + 0.41 * (%G+C) - 675/n wherein [Na+] is the molar salt concentration; [K+] = [Na+] and n = number of bases in the oligonucleotide .Step B according to Claim 1 is carried out in the second chamber.

Upon annealing of the primer oligonucleotides the reaction mixture is passed to the third reaction chamber whose temperature is regulated to that suitable for the action of the polymerase used in the method. In the case of the commonly used Taq polymerase this would be 74°C.

As taught by Kim and Smithies (Nucleic Acids Research 16, 8887-8903) incubation at the intermediate "extension" temperature is unnecessary. Accordingly only two temperatures are required one in the range from 37-72.degree. C. for annealing and extension and one in the 85-98.degree. C. range for denaturation. Therefore, a further embodiment of Device 1 may consist of only three chambers wherein chambers 2 and 3 are replaced by a single chamber, said chamber further comprising a solid support upon which the polymerase is immobilised.

Subsequent to primer extension the reaction mixture is passed to a fourth reaction chamber for Steps D and E. Said reaction chamber further comprises a solid phase upon which a methyltransferase is immobilised. At this stage the required co factor for the methyltransferase (e.g. S-adenosylmethionine) is added to the reaction solution. The reaction mixture is passed through the reaction chamber such that the hemimethylated double stranded nucleic acids are contacted with the methyltransferase enzyme sufficient for the methylation of all nucleic acids within the reaction mixture. Reaction times and conditions are calculated and optimised according to the methyltransferase as well as CpG composition of the amplificate nucleic acids. The reaction solution is then passed back to the first reaction chamber allowing Steps A-E to be repeated until the desired quantity of amplificates is synthesised.

In its simplest form, the thermostatic means described in the inventions above may comprise two metal block heat exchangers separated by a layer of insulation. The heat exchangers could also be other types of heat exchangers such as thermostatically controlled constant temperature fluid baths. Each metal block heat exchanger is preferably made of aluminium or some other good heat conducting metal to minimise temperature gradients therein.

The temperature of each metal block is maintained at a constant temperature by any suitable temperature control system. A suitably programmable control system which may be used is disclosed in U.S. Pat. No. 5,038,852 and in U.S. Patent application No. 07/871,264, filed Apr. 20, 1992.

Peltier devices are ideal for controlling the temperatures of the metal blocks because these metal block heat exchangers are each maintained at a constant temperature. Suitable known temperature sensing and feedback control circuits (not shown) are necessary to control the direction of current flow through the Peltier devices to maintain the block temperature constant by extracting heat from the block when it gets too hot and adding heat when it gets too cold. Any other temperature control system will also work for the blocks such as resistance heaters and/or heated/chilled fluid circulating through passages in the metal blocks with the chilled fluid being, for example, tap water or antifreeze chilled by circulating Freon of a refrigeration unit, depending upon the desired temperatures of the blocks.

Sample handling and reaction conditions, including, flow, velocity, pressure, and temperature are all controlled according to a user defined protocol by a computer (5). Connections between said computer and components of the device illustrated by, but not limited to, dotted lines.

### Single reaction chamber methylation retaining PCR device

In a further embodiment of a methylation retaining PCR device (hereinafter referred to as Device 2), all steps of the method are carried out in one reaction chamber. The device comprises a thermocycling reaction chamber (10), two vessels (7) containing reagents (8) required for the different stages of the procedure. The reaction chamber is thermostatically cycled between temperatures suitable for Steps A, B, C, D and E of the reaction as described above.

In said embodiment the apparatus comprises 2 vessels (7) and a reaction chamber (10). The vessels are each connected to the reaction chamber by tube means (11), reaction solution is passed through the tubes by means of a pump (12), preferably a peristaltic pump. The tubes further comprise valves (9) ensuring that the reaction solution flows in a unidirectional manner. A first vessel contains all reagents necessary for Steps A to C according to Claim 1 for example, but not limited to, dNTPs, reaction buffer and polymerase. A second vessel contains all reagents necessary for Steps D and E for example, but not limited to, reaction buffer, methyltransferase and cofactors. The reaction chamber is a thermocycling vessel comprising a solid surface (13) upon which an excess of primer oligonucleotides required during Step B of the reaction are immobilised. Sample DNA and reagents from the first vessel are passed into the reaction chamber where the denaturation, annealing and extension processes (Steps A-C) are carried out. Primer oligonucleotides immobilised upon a solid surface hybridise to a genomic DNA during Step B of the first cycle of the reaction. Single stranded nucleic acid molecules generated by Step A of the reaction hybridise to the primer oligonucleotides. After extension (Step C), methylation (Steps D and E) and denaturation (Step A) the single stranded template nucleic acids hybridise to unused primer oligonucleotides in the vicinity of the primer oligonucleotides to which they were hybridised in the previous cycle.

Upon completion of the extension (Step C) and prior to the methylation reaction (Steps D and E) the reaction mixture is drained from the reaction chamber by means of an outlet (15). Said outlet may further comprise a valve and pump mechanism (12) as illustrated. Reagents from the second vessel are passed into the reaction chamber and methylation of the hemimethylated nucleic acids is carried out under appropriate conditions. The reaction chamber may then be drained of reagents allowing reagents from the first vessel to be passed into the reaction chamber and allowing Steps A to D of the reaction to proceed again until the desired quantity of methylated nucleic acids is synthesised.

Sample handling and reaction conditions, including, flow, velocity, pressure, and temperature are all controlled according to a user defined protocol by a computer (14). Connections between said computer and components of the device illustrated by, but not limited to, dotted lines.

In one embodiment of the invention (e.g. Device 1) the polymerase and methylase enzymes as used in Steps C, D and E of the reaction may be immobilised upon a solid support. In a further embodiment (e.g. Device 2) primer oligonucleotides as utilised in Step C of the method are required to be immobilised upon a solid support. The solid support may be beads, particles, sheets, dipsticks, rods, membranes, filters, fibres (e.g., optical and glass), and suchlike. Preferably, the solid support is a bead. The material composition of the solid support includes, but is not limited to, polystyrene, nitrocellulose, plastic, nylon, glass, silica, metal, metal alloy, polyacrylamide, polyacrylate, crosslinked-dextran and combinations thereof. Preferably, the solid support is thermally stable (e.g., able to withstand temperatures of up to 100.degree. C.) to withstand thermocycling conditions as described in the invention. Preferably, the solid support is capable of being modified by the attachment of oligonucleotide primers. Methods for the immobilisation of oligonucleotides are known in the art and include the use of photolabile groups and solid phase chemistry (U.S. Patent 5,744,305). Methods for the immobilisation of enzymes are also well known in the art for example in 'Immobilization of Enzymes and Cells' Bickerstaff and Walker, Humana Press 1996.

### Double reaction chamber methylation retaining PCR device

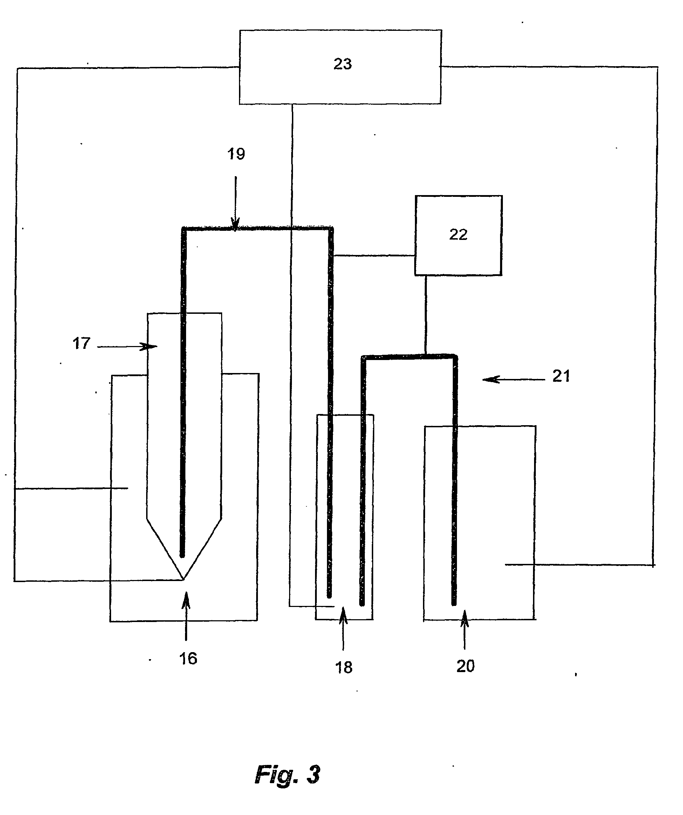

A third embodiment of a methylation retaining PCR device is illustrated in Figure 3, said embodiment is hereinafter referred to as Device 3. The illustrated embodiment of the device has two reaction chambers. A first reaction chamber comprises a thermocycling unit (16) and a vessel (17) for containing the reaction solution. Steps A to C of the reaction are carried out in this chamber. The reaction solution is then pumped from the first reaction chamber to a second reaction chamber (18) using tube means (19) wherein Step D of the method is carried out. Reagents required for Step D of the reaction are contained in a vessel (20) which is connected to reaction chamber (18) by tube means. Reagents from the vessel (20) are pumped into reaction chamber (18) by tube means (21). The pump (22) may take in form standard in the art, for example a peristaltic pump. However, particularly preferred is a syringe pump.

Sample handling and reaction conditions, including, flow, velocity, pressure, and temperature are all controlled according to a user defined protocol by a computer (23). Connections between said computer and components of the device illustrated by, but not limited to, dotted lines.

Thermocycling units suitable for use in nucleic acid amplification techniques are known in the art and commercially available, for example, from manufacturers such as Perkins Elmer and Eppendorf.

### Figures

### Figure 1

Figure 1 illustrates a multiple reaction chamber methylation retaining PCR device.

### Figure 2

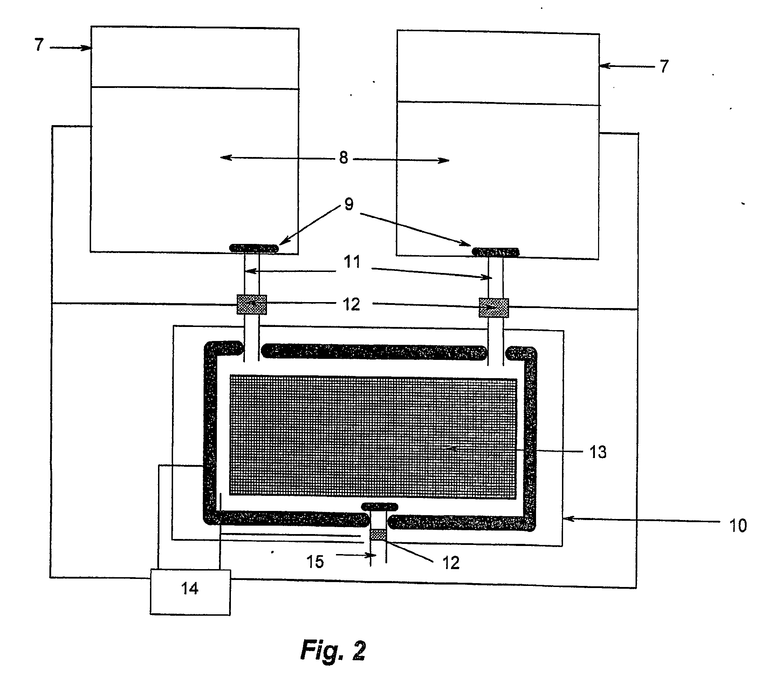

Figure 2 illustrates a single reaction chamber methylation retaining PCR device.

### Figure 3

Figure 3 illustrates a double reaction chamber methylation retaining PCR device.

### Examples

### Example 1

Genomic DNA commercially available from Promega is used in the analysis. A CpG rich fragment of the regulatory region of the GSTPi gene is used in the analysis. The DNA is firstly artificially methylated at all cytosine 5 positions within the CpGs (upmethylation). The upmethylated DNA is then amplified using one round of PCR. The resultant amplificate is then divided into two samples, Sample A (the control sample) is amplified using conventional PCR. Sample B is amplified according to the disclosed method. The two samples are then compared in order to ascertain the presence of methylated CpG positions within Sample B. The comparison is carried out by means of a bisulphite treatment and analysis of the treated nucleic acids.

### Upmethylation

### Reagents:

### DNA

SssI Methylase (concentration 2 units/µl).
SAM (S-adenosylmethionine)
4,5 µl Mss1-Buffer (NEB Buffer B+ (10 mM Tris-HCl 300 mM NaCl, 10 mM Tris-HCl, 0.1 mM EDTA, 1 mM dithiothreitol, 500 µg/ml BSA, 50% glycerol (pH 7.4 at 25°C)pH 7.5; 10 mM MgCl₂; 0,1 mg/ml BSA)
dd water (0.2 µm-filtered autoclaved, DNases, RNases, proteases, phosphatases-free).

### Method:

Reagents are combined and incubated at 37 degrees for 16 hours. The sample may then be stored in the refrigerator (+4 °C).

The upmethylated DNA is digested using the restriction enzyme

### PCR

### Reagents:

primer I : TTCGCTGGAGTTTCGCC
primer II :GCTTGGGGGAATAGGGAG
HotStart Taq Polymerase (QIAGEN)
10 x PCR buffer (QIAGEN)
dNTP solution (25·mM each)
water (0.2 µm-filtered, autoclaved, DNases, RNases, proteases, phosphatases-free).

Reagents are to be combined in a reaction solution in the order above. The reaction solution is then cycled in a thermalcycler according to the following. An initial denaturation at 95°C is carried out for 15 min. This is followed by primer annealing at 55°C for 45 sec. and elongation at 72°C for 1.5 min.

The resultant reaction solution is then divided into two equal samples, A and B. Each sample is treated as below.

### Sample A

Standard PCR as described above. The reaction is cycled for 40 cycles at 95°C for 1 minute, 55°C for 45 sec. and elongation at 72°C for 1.5 min.

### Sample B

### Reagents:

Human DNA (cytosine-5) Methyltransferase (New England Biolabs)
Dnmt 1 reaction buffer (50 mM TrisHCL pH7.8, 1 mM EDTA, 1 MM dithiothreitol, 7 µg/ml PMSF, 5% glycerol)
100 µg/ml BSA

### Steps 1 to 4 are repeated 40 times:

1. DNA is precipitated and pelleted, resuspended using Dnmt1 reaction buffer, DNMT and BSA .
2. The reaction solution is incubated at 37°C.
3. DNA is precipitated and pelleted, resuspended using PCR reagents as above.
4. One cycle of PCR is carried out at 95°C for 1 minutes, 55°C for 45 sec. and elongation at 65°C for 2 min.

### Sample Analysis

Both samples are analysed in order to ascertain their relative levels of methylation. In a first step the two samples are treated in order to distinguish between methylated and non methylated cytosines. The treatment is carried out using a solution of sodium-disulfite. The treatment converts cytosine to thymine while preserving 5-methyl-cytosine as cytosine. Sample A is thereby thymine rich relative to Sample B, which is relatively cytosine rich. Following bisulphite treatment both samples are analysed by means of sequencing in order to ascertain their degree of methylation (i.e. relative concentrations of cytosine and thymine). Sequencing is carried out by means of the Sanger method using the ABI 310 sequencer (Applied Biosystems).

## Claims

1. A method for the amplification of genomic DNA whereby the cytosine methylation pattern of the genomic DNA is retained in the amplificate sequence(s), said method comprising the following steps:
(a) heating the genomic DNA to a temperature operative to cause denaturation
(b) cooling the denatured DNA in the presence of single stranded oligonucleotide primers such that the primers anneal to the DNA
(c) heating the mixture in the presence of a polymerase and nucleotides to a temperature such that the primers are extended resulting in a hemimethylated double stranded nucleic acid
(d) contacting the double stranded nucleic acid with a maintenance methyltransferase and a methyl donor molecule
(e) enzymatically methylating the hemimethylated double stranded DNA according to the methylation status of the CpG dinucleotides within the template strand
(f) repeating steps A-E a desired number of times to reach a desired number of nucleic acids.

2. A method according to Claim 1 wherein
the methyltransferase is DNA (cytosine-5) Methyltransferase (DNMT 1).

3. A method according to Claims 1 or 2 wherein the methyl donor molecule is S-adenosylmethionine.

4. A method according to Claims 1 to 3 wherein the methyl group carries a detectable label which is incorporated into the synthesised nucleic acid strand.

5. A method according to Claims 1 to 4 wherein the methyltransferase is immobilised on a solid surface.

6. A method according to Claim 1 further comprising Step (g) a treatment with an agent capable of distinguishing between methylated and unmethylated cytosine bases.

7. A method according to Claim 6 wherein the agent is a methylation sensitive restriction enzyme.

8. A method according to Claim 6 wherein the agent is a bisulphite solution.

9. Use of a device comprising two or more reaction chambers, channel means providing fluid connections between adjacent chambers and the first and last reaction chambers, temperature regulating means for controlling the temperature of each reaction chamber for the methylation pattern retaining amplification of nucleic acids according to Claim 1.

10. Use of a device comprising
two vessels,
a reaction chamber,
temperature regulating means for controlling the temperature of the reaction chamber,
means for transferring liquid reagent form the first and second vessels to the reaction chamber,
channel means providing fluid connections between adjacent chambers and the first and last reaction chambers
means for draining liquid reagents from the reaction chamber
for the methylation pattern retaining amplification of nucleic acids according to Claim 1 to 4.

## Patentansprüche

1. Verfahren zur Amplifikation von genomischer DNA, wodurch das Cytosin-Methylierungsmuster der genomischen DNA in der Amplifikat-Sequenz beziehungsweise den Amplifikat-Sequenzen erhalten bleibt, wobei das genannte Verfahren die folgenden Schritte umfasst :
(a) Erhitzen der genomischen DNA auf eine Temperatur, die wirksam ist, Denaturierung zu verursachen,
(b) Kühlen der denaturierten DNA in Anwesenheit einzelsträngiger Oligonukleotid-Primer, so dass die Primer an die DNA assozieren,
(c) Erhitzen der Mischung in Anwesenheit einer Polymerase und von Nukleotiden auf eine Temperatur, so dass die Primer verlängert werden, was in einer hemimethylierten doppelsträngigen Nukleinsäure resultiert,
(d) in Kontakt Bringen der doppelsträngigen Nukleinsäure mit einer Erhaltungs-Methyltransferase und einem Methyldonor-Molekül,
(e) enzymatische Methylierung der hemimethylierten doppelsträngigen DNA gemäß dem Methylierungsstatus der CpG-Dinukleotide innerhalb des Templat-Strangs,
(f) Wiederholen der Schritte A-E für eine gewünschte Anzahl von Malen, um eine gewünschte Anzahl von Nukleinsäuren zu erreichern.

2. Verfahren gemäß Anspruch 1, worin die Methyltransferase DNA-(Cytosin-5)-Methyltransferase (DNMT 1) ist.

3. Verfahren gemäß den Ansprüchen 1 oder 2, worin das Methyldonor-Molekül S-Adenosylmethionin ist.

4. Verfahren gemäß den Ansprüchen 1 bis 3, worin die Methylgruppe einen detektierbaren Marker trägt, der in den synthetisierten Nukleinsäure-Strang inkorporiert ist.

5. Verfahren gemäß den Ansprüchen 1 bis 4, worin die Methyltransferase auf einer festen Oberfläche immobilisiert ist.

6. Verfahren gemäß Anspruch 1, das weiterhin Schritt (g), die Behandlung mit einem Mittel, das zur Unterscheidung zwischen methylierten und unmethylierten Cytosin-Basen geeignet ist, umfasst .

7. Verfahren gemäß Anspruch 6, worin das Mittel ein methylierungssensitives Restriktionsenzym ist.

8. Verfahren gemäß Anspruch 6, worin das Mittel eine Bisulfitlösung ist.

9. Verwendung einer Vorrichtung, die zwei oder mehr Reaktionskammern, Verbindungswege, welche die Flüssigkeitsverbindungen zwischen benachbarten Kammern und der ersten und letzten Reaktionskammer bereitstellen, Temperaturregulierungsvorrichtungen zur Kontrolle der Temperatur einer jeden Reaktionskammer umfasst, für die Methylierungsmuster-erhaltende Amplifikation von Nukleinsäuren gemäß Anspruch 1.

10. Verwendung einer Vorrichtung, umfassend
zwei Behälter,
eine Reaktionskammer,
Temperaturregulierungsvorrichtung zur Kontrolle der Temperatur der Reaktionskammer,
Vorrichtung, um das flüssige Reagens vom ersten und zweiten Behälter in die Reaktionskammer zu überführen,
Verbindungswege, welche für die Flüssigkeitsverbindungen zwischen benachbarten Kammern und der ersten und letzten Reaktionskammer bereitstellen, Vorrichtung zum Ablassen der flüssigen Reagenzien aus der Reaktionskammer,
zur Methylierungsmuster-erhaltenden Amplifikation von Nukleinsäuren gemäß Anspruch 1 bis 4.

## Revendications

1. Procédé pour amplifier de l'ADN génomique, moyennant quoi le schéma de méthylation de la cytosine de l'ADN génomique est conservé dans la (les) séquence (s) d'amplification, ledit procédé comprenant les étapes suivantes :
(a) chauffer l'ADN génomique jusqu'à une température valable pour provoquer une dénaturation
(b) refroidir l'ADN dénaturé en présence d'amorces oligonucléotidiques en simple brin, tel que les amorces s'associent à l'ADN
(c) chauffer le mélange en présence d'une polymérase et de nucléotides jusqu'à une température telle que les amorces soient allongées, ce qui conduit à un acide nucléique hémiméthylé en double brin
(d) mettre en contact l'acide nucléique en double brin avec une méthyltransférase d'entretien et une molécule donneuse de méthyle
(e) méthyler enzymatiquement l'ADN hémiméthylé en double brin, selon l'état de méthylation des dinucléotides CpG se trouvant dans le brin matrice
(f) répéter, le nombre souhaité de fois, les étapes A à E pour atteindre le nombre souhaité d'acides nucléiques.

2. Procédé selon la revendication 1, dans lequel la méthyltransférase est une ADN (cytosine-5) méthyltransférase (DNMT 1).

3. Procédé selon les revendications 1 ou 2, dans lequel la molécule donneuse de méthyle est une S-adénosylméthionine.

4. Procédé selon les revendications 1 à 3, dans lequel le groupe méthyle transporte un marqueur détectable qui est incorporé dans le brin d'acide nucléique synthétisé.

5. Procédé selon les revendications 1 à 4, dans lequel la méthyltransférase est immobilisée sur une surface solide.

6. Procédé, selon la revendication 1, comprenant en outre une étape (g) : un traitement avec un agent capable de faire une distinction entre des bases de cytosine méthylées et non méthylées.

7. Procédé selon la revendication 6, dans lequel l'agent est une enzyme de restriction sensible à la méthylation.

8. Procédé selon la revendication 6, dans lequel l'agent est une solution de bisulfite.

9. Utilisation d'un appareil comprenant : deux ou plusieurs chambres de réaction ; un moyen à canaux fournissant des connexions liquides entre des chambres adjacentes et la première et la dernière chambres ; un moyen de régulation de la température destiné à contrôler la température de chaque chambre de réaction pour l'amplification, qui conserve le schéma de méthylation, d'acides nucléiques selon la revendication 1.

10. Utilisation d'un appareil comprenant :
deux récipients ;
une chambre de réaction ;
un moyen de régulation de la température destiné à contrôler la température de la chambre de réaction ;
un moyen pour transférer un réactif liquide à partir des premier et deuxième récipients vers la chambre de réaction ;
un moyen à canaux fournissant des connexions liquides entre des chambres adjacentes et la première et la dernière chambres de réaction ;
un moyen pour drainer des réactifs liquides à partir de la chambre de réaction ;
pour l'amplification, qui conserve le schéma de méthylation, d'acides nucléiques selon les revendications 1 à 4.
